# EUROPÄISCHE PATENTSCHRIFT

(11) **EP 0 526 493 B1**
(45) Veröffentlichungstag und Bekanntmachung des Hinweises auf die Patenterteilung: **13.07.1994**
(21) Anmeldenummer: 91907808.9
(22) Anmeldetag: 23.04.1991
(51) Int. Cl.: A61N 1/08, A61N 1/36

(54) **VERFAHREN ZUM BETREIBEN EINER ANORDNUNG ZUR GEWEBESTIMULATION**
METHOD OF OPERATING A TISSUE STIMULATING DEVICE
PROCEDE DE COMMANDE D'UN STIMULATEUR DE TISSUS

(30) Priorität: 24.04.1990 DE 4013048
(43) Veröffentlichungstag der Anmeldung: 10.02.1993
(73) Patentinhaber: Pacesetter AB, 171 95 Solna (SE)
(72) Erfinder: HOLMSTRÖM, Nils, S-175 45 Järfälla (SE)
(74) Vertreter: Lettström, Richard Wilhelm
(86) Internationale Anmeldenummer: EP9100779
(87) Internationale Veröffentlichungsnummer: WO9116102

(56) Entgegenhaltungen:
- EP-A- 0 334 675
- DE-B- 2 254 928
- US-A- 3 759 265
- US-A- 4 290 430
- US-A- 4 402 322

## Beschreibung

Die Erfindung betrifft ein Verfahren zum Betreiben einer Anordnung zur Gewebestimulation bei einem Lebewesen mit einem aus einer Spannungsquelle gespeisten Stimulationsimpulsgenerator zur Erzeugung von Stimulationsimpulsen, mit einer Detektoreinrichtung zur Erfassung der Reaktion des Gewebes auf die Stimulation und mit einer den Stimulationsimpulsgenerator steuernden Steuereinrichtung, die zur Ermittlung der Stimulationsempfindlichkeit des Gewebes solange eine Veränderung der Energie nacheinander erzeugter Stimulationsimpulse veranlaßt, bis die Detektoreinrichtung einen Wechsel von einem Ausbleiben der Reaktion zu einer Reaktion oder umgekehrt erfaßt, wobei anschließend die Stimulationsimpulse mit einer Energie erzeugt werden, die dem für die Stimulationsempfindlichkeit ermittelten Wert zuzüglich einem Sicherheitswert entspricht.

Bei einer derartigen, aus der US-PS 4,878,497 bekannten Anordnung handelt es sich um einen Herzschrittmacher, mit dem das Herz eines Patienten mit Hilfe von Stimulationsimpulsen angeregt wird. Dabei erfolgt eine Kontraktion als Reaktion des Herzens nur dann, wenn die Energie der Stimulationsimpulse eine bestimmte, sich aus der jeweiligen Stimulationsempfindlichkeit des Herzgewebes ergebende Reizschwelle überschreitet. Um den Energieverbrauch bei dem bekannten Herzschrittmacher gering zu halten, wird die Stimulationsenergie an die veränderbare Stimulationsempfindlichkeit angepaßt, indem in bestimmten Zeitabständen die Stimulationsempfindlichkeit des Herzgewebes ermittelt wird und anschließend die Stimulationsenergie auf einen Wert eingestellt wird, der dem für die Stimulationsempfindlichkeit ermittelten Energiewert (Reizschwelle) zuzüglich einem Sicherheitswert entspricht. Zur Ermittlung der Stimulationsempfindlichkeit wird die Energie der Stimulationsimpulse ausgehend von einem unter der Reizschwelle liegenden Wert nacheinander schrittweise solange erhöht, bis mittels einer Detektoreinrichtung eine Reaktion des Herzgewebes auf die Stimulation detektiert wird. Nach der Ermittlung der Stimulationsempfindlichkeit und der daran angepaßten Einstellung der Stimulationsenergie wird bis zu dem nächsten Ermittlungsvorgang durch Detektion der Herzreaktion regelmäßig überprüft, ob die eingestellte Stimulationsenergie die Reizschwelle des Herzgewebes übersteigt. Ist dies nicht der Fall, so wird die Stimulationsenergie erhöht. Dabei und bei der Ermittlung der Stimulationsempfindlichkeit erfolgt die Änderung der Energie der Stimulationsimpulse in der Weise, daß ausgehend von einer momentanen Impulsdauer zunächst die Impulsamplitude (elektrische Stromstärke) schrittweise bis auf einen bestimmten Wert erhöht wird, anschließend die Impulsdauer schrittweise vergrößert wird und schließlich bei erreichen einer maximalen Impulsdauer die Impulsamplitude auf einen Maximalwert erhöht wird.

Aus der DE-AS 22 54 928 ist ein Herzschrittmacher bekannt, bei dem zur Ermittlung der Stimulationsempfindlichkeit des Herzgewebes die Impulsamplitude aufeinanderfolgender Stimulationsimpulse ausgehend von einem über der Reizschwelle des Herzgewebes liegenden Wert schrittweise verringert wird, bis ein Ausbleiben der Reaktion des Herzens detektiert wird.

Bekanntlich weisen von denjenigen Stimulationsimpulse, die bei einer bestimmten Stimulationsempfindlichkeit eines zu stimulierenden Gewebes gerade noch in der Lage sind, eine Stimulation auszulösen, diejenigen Stimulationsimpulse mit kürzerer Impulsdauer und höherer Impulsamplitude einen geringeren Stromverbrauch auf, als Stimulationsimpulse mit geringerer Impulsamplitude und längerer Impulsdauer. Da insbesondere bei implantierten Anordnungen die Stimulationsenergie in der Regel aus einer Batterie als Spannungsquelle bezogen wird, wird in Verbindung mit der Erzeugung der Stimulationsimpulse eine möglichst geringe Ladungsentnahme aus der Spannungsquelle angestrebt. Es besteht ferner, insbesondere bei Ärzten der Wunsch, den Sicherheitswert zwischen der Reizschwelle des Gewebes und der in Abhängigkeit davon eingestellten Stimulationsenergie als elektrische Spannung, d.h. in Volt ausdrücken zu können.

Gemäß der Erfindung ist vorgesehen, daß bei der Anordnung der eingangs angegebenen Art bei der Ermittlung der Stimulationsempfindlichkeit die Stimulationsimpulse mit einer Impulsamplitude erzeugt werden, deren Spannung um einen dem Sicherheitswert entsprechenden Spannungswert unter der maximal verfügbaren Spannung der Spannungsquelle liegt, wobei zur Veränderung der Stimulationsenergie die Impulsdauer der Stimulationsimpulse verändert wird, und daß im Anschluß an die Ermittlung der Stimulationsempfindlichkeit die Stimulationsimpulse mit einer der verfügbaren Spannung der Spannungsquelle entsprechenden Impulsamplitude und der bei der Detektion des Wechsels bei der Reaktion des Gewebes eingestellten Impulsdauer erzeugt werden. Dadurch, daß in der Zeitdauer zwischen jeweils zwei Vorgängen zur Ermittlung der Stimulationsempfindlichkeit Stimulationsimpulse mit einer der verfügbaren Spannung der Spannungsquelle entsprechenden Impulsamplitude erzeugt werden, ist die Strom- bzw. Ladungsentnahme aus der Spannungsquelle so gering wie möglich. Dabei liegt die Impulsamplitude in vorteilhafter Weise um den als elektrische Spannung, d.h, in Volt ausgedrückten Sicherheitswert über der zuletzt ermittelten Reizschwelle des zu stimulierenden Gewebes, so daß hiermit für den behandelnden Arzt eine sichere Information über den Sicherheitswert gegeben ist.

Um zu verhindern, daß in dem Zeitraum zwischen jeweils zwei aufeinanderfolgenden Ermittlungen der Stimulationsempfindlichkeit die Reizschwelle des zu stimulierenden Gewebes aufgrund einer Abnahme der Stimulationsempfindlichkeit über den eingestellten Wert für die Stimulationsenergie steigt, ist entsprechend einer vorteilhaften Weiterbildung der erfindungsgemäßen Anordnung vorgesehen, daß nach der Ermittlung der Stimulationsempfindlichkeit bis zur nächsten Ermittlung in sich wiederholenden vorgegebenen Zeitabständen einzelne Stimulationsimpulse mit um den Sicherheitswert verringerter Impulsamplitude erzeugt werden, daß dabei die Reaktion des Gewebes mittels der Detektoranordnung detektiert wird und daß bei einem Ausbleiben der Reaktion für die nächsten Stimulationsimpulse die Impulsdauer erhöht und die Impulsamplitude auf den der verfügbaren Spannung entsprechenden Wert zurückgestellt wird. Auf diese Weise wird auch bei einer unvorhersehbar schnellen Abnahme der Stimulationsempfindlichkeit stets eine sichere Stimulation ohne erfolglose Stimulationsversuche erreicht.

Im einfachsten Fall kann vorgesehen werden, daß bei Detektion einer Reaktion des Gewebes auf den jeweiligen einzelnen Stimulationsimpuls die Impulsamplitude für die nächsten Stimulationsimpulse bei unveränderter Impulsdauer auf den der verfügbaren Spannung entsprechenden Wert zurückgestellt wird. Um auch während der Zeitdauer zwischen jeweils zwei Ermittlungen der Stimulationsempfindlichkeit eine energiesparende Anpassung der Stimulationsenergie an die Stimulationsempfindlichkeit zu erreichen, wird in vorteilhafter Weise bei Detektion einer Reaktion des Gewebes auf den jeweils einzelnen Stimulationsimpuls die Impulsdauer für die nächsten Stimulationsimpulse vermindert und die Impulsamplitude auf den der verfügbaren Spannung entsprechenden Wert zurückgestellt.

Wird für die Stimulation und die Detektion der Stimulationsantwort (Reaktion) des Gewebes eine gemeinsame Stimulationselektrode verwendet, so wird die Stimulationsantwort von durch die Stimulation in dem Gewebe verursachten Polarisationserscheinungen eine zeitlang bis zur Unkenntlichkeit überlagert. In diesem Zusammenhang ist aus der DE-OS 23 42 030 die Erzeugung von bipolaren Stimulationsimpulsen aus einem ersten, die Stimulation auslösenden Teilimpuls und einem diesem unmittelbar folgenden zweiten Teilimpuls entgegengesetzter Polarität bekannt, wobei der Energieinhalt des zweiten Teilimpulses derart bemessen ist, daß die von dem ersten Teilimpuls im Gewebe erzeugten Polarisationserscheinungen so schnell abgebaut werden, daß anschließend eine störungsfreie Erfassung der Stimulationsantwort möglich ist. Der Energieverbrauch von biphasischen Stimulationsimpulsen ist jedoch etwa doppelt so groß wie der Energieverbrauch von monophasischen Stimulationsimpulsen mit gleicher Wirkung für die Auslösung einer Stimulation. Um daher den Energieverbrauch so gering wie möglich zu halten, ist entsprechend einer Weiterbildung der erfindungsgemäßen Anordnung vorgesehen, daß bei einem Anschluß des Stimulationsimpulsgenerators und der Detektoreinrichtung an einer gemeinsamen Stimulationselektrode, die in Verbindung mit einer nachfolgenden Detektion der Reaktion des Gewebes zu erzeugenden Stimulationsimpulse in Form von biphasischen Stimulationsimpulsen erzeugt werden und daß alle übrigen Stimulationsimpulse in Form von monophasischen Impulsen erzeugt werden.

Dabei besteht jeder biphasischer Stimulationsimpuls vorteilhafterweise aus Teilimpulsen unterschiedlicher Polarität mit einer zwischen den Teilimpulsen liegenden Impulspause, wobei die Teilimpulse jeweils eine weitgehend gleiche Impulsamplitude und Impulsdauer aufweisen. Durch die Impulspause zwischen den Teilimpulsen wird nämlich verhindert, daß die Wirksamkeit des ersten Teilimpulses für die Auslösung einer Stimulation durch den darauffolgenden Teilimpuls reduziert wird. Es konnte festgestellt werden, daß das zu stimulierende Gewebe auf die von der erfindungsgemäßen Anordnung erzeugten biphasischen Stimulationsimpulse etwa mit der gleichen Stimulationsempfindlichkeit, bei größeren Impulspausen sogar mit erhöhter Stimulationsempfindlichkeit (d.h. niedrigerer Reizschwelle) reagiert, als auf dem jeweils ersten Teilimpuls entsprechende monophasische Stimulationsimpulse.

Wie eingangs bereits erwähnt, ist der Stromverbrauch bei Stimulationsimpulsen mit kürzerer Impulsdauer und höherer Impulsamplitude geringer als bei Stimulationsimpulsen mit geringer Impulsamplitude und längerer Impulsdauer. Es ist jedoch insbesondere bei einer Detektion der Reaktion des Gewebes auf die Stimulation erforderlich, die Impulsdauer auf einen maximalen Wert zu begrenzen, um die Detektion überhaupt zu ermöglichen. Für diesen Fall ist gemäß einer vorteilhaften Ausbildung der erfindungsgemäßen Anordnung vorgesehen, daß zur Veränderung der Energie der Stimulationsimpulse deren Impulsdauer ausgehend von der durch die verfügbare Spannung der Spannungsquelle vorgegebenen Impulsamplitude zwischen einem Minimalwert und einem Maximalwert verändert wird und daß bei Erreichen des Maximalwertes zur Einstellung einer noch höheren Energie die Impulsamplitude auf einen durch Vervielfachung der Spannung der Spannungsquelle erhöhten Wert eingestellt wird.

Zur Erläuterung der Erfindung wird im Folgenden auf die Figuren der Zeichnung Bezug genommen; im einzelnen zeigen
- FIG 1: ein Blockschaltbild eines Ausführungsbeispiels der erfindungsgemäßen Anordnung,
- FIG 2: in einem Zeitdiagramm einen biphasischen und einen monophasischen Stimulationsimpuls sowie zu ihrer Erzeugung erforderliche Steuersignale, die
- FIG 3 und 4: jeweils ein Beispiel für den zeitlichen Verlauf der Stimulationsempfindlichkeit eines zu stimulierenden Gewebes und den Verlauf der daran angepaßten Stimulationsenergie und
- FIG 5: in einem Diagramm die bei einer bestimmten Stimulationsempfindlichkeit zur Gewebestimulation mindestens erforderliche Spannung und Ladung eines Stimulationsimpulses in Abhängigkeit von seiner Impulsdauer.

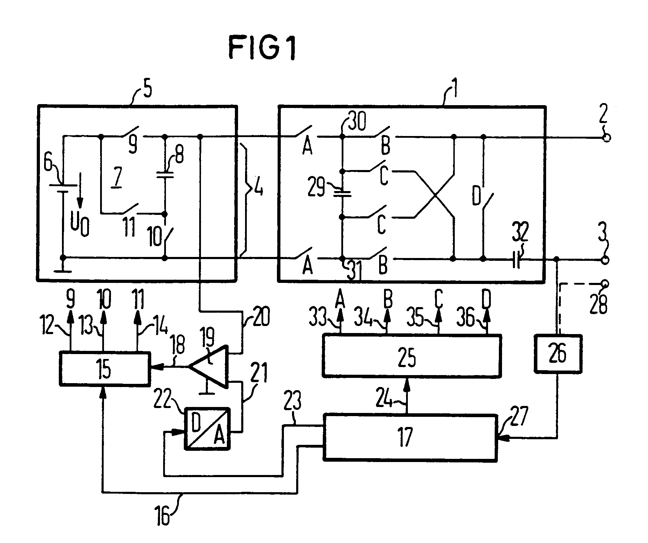

FIG 1 zeigt als Ausführungsbeispiel für die erfindungsgemäße Anordnung das Blockschaltbild eines Herzschrittmachers zur Gewebestimulation, in diesem Fall der Stimulation eines Herzens. Der Herzschrittmacher enthält einen Stimulationsimpulsgenerator (1), an dem ausgangsseitig eine indifferente Elektrode (2) und eine Stimulationselektrode (3) angeschlossen ist. Die indifferente Elektrode (2) wird von dem hier nicht dargestellten Gehäuse des Herzschrittmachers gebildet, während die Stimulationselektrode (3) in dem Herzen plaziert ist. Der Stimulationsimpulsgenerator (1) ist eingangsseitig an dem Ausgang (4) eines Stimulationsspannungsgenerators (5) angeschlossen, der den Stimulationsimpulsgenerator (1) mit einer vorgebbaren Spannung für die zu erzeugenden Stimulationsimpulse versorgt.

Der Stimulationsimpulsgenerator (5) enthält eine Batterie (6) mit einer Batteriespannung U₀ und eine Spannungsverdoppelungsschaltung (7), die aus einem Kondensator (8) und drei einzeln steuerbaren Schaltern (9,10,11) besteht. Die Batterie (6) ist über den mit (9) bezeichneten Schalter direkt an den Ausgang (4) des Stimulationsspannungsgenerators (5) schaltbar. Der Kondensator (8) ist über denselben Schalter (9) und den Schalter (10) parallel mit der Batterie (6) verbindbar oder bei geöffneten Schaltern (9,10) und geschlossenem Schalter (11) in Reihenschaltung mit der Batterie (6) an dem Ausgang (4) des Stimulationsspannungsgenerators (5) angeschlossen. Die Schalter (9,10,11) werden einzeln über ihnen zugeordnete Steuerleitungen (12,13,14) eines Schaltimpulsgenerators (15) gesteuert, der seinerseits an einer ersten ausgangsseitigen Steuerleitung (16) einer Steuereinrichtung (17) angeschlossen ist. Der Schaltimpulsgenerator (15) ist über eine zusätzliche Steuerleitung (18) mit dem Ausgang eines Komparators (19) verbunden, der über einen ersten Eingang (20) an dem Ausgang (4) des Stimulationsspannungsgenerators (5) und über einen zweiten Eingang (21) an dem Ausgang eines Digital-/Analog-Umsetzers (22) angeschlossen ist, der seinerseits eingangsseitig über eine zweite ausgangsseitige Steuerleitung (23) der Steuereinrichtung (17) an diese angeschlossen ist. Die Steuereinrichtung (17) gibt den Wert für die von dem Stimulationsspannungsgenerator (5) zu erzeugende Spannung vor und veranlaßt durch den Schaltimpulsgenerator (15) eine entsprechende Steuerung der Schalter (9,10,11), wie dies untenstehend noch erläutert wird.

Die Steuereinrichtung (17) ist ferner über eine dritte ausgangsseitige Steuerleitung (24) mit einer Zeitgebereinrichtung (25) verbunden, die, wie nachstehend noch erläutert wird, den zeitlichen Verlauf der Erzeugung der Stimulationsimpulse in dem Stimulationsimpulsgenerator (1) steuert. An der Stimulationselektrode (3) ist eine Detektoreinrichtung (26) angeschlossen, die nach Abgabe eine Stimulationsimpulses durch den Stimulationsimpulsgenerator (1) die Reaktion des Gewebes erfaßt und ausgangsseitig mit einem Steuereingang (27) der Steuereinrichtung (17) verbunden ist. Die Detektoreinrichtung (26) kann anstelle mit der Stimulationselektrode (3) auch mit einer in deren Nähe angeordneten Meßelektrode (28) verbunden sein, wie dies in FIG 1 gestrichelt dargestellt ist.

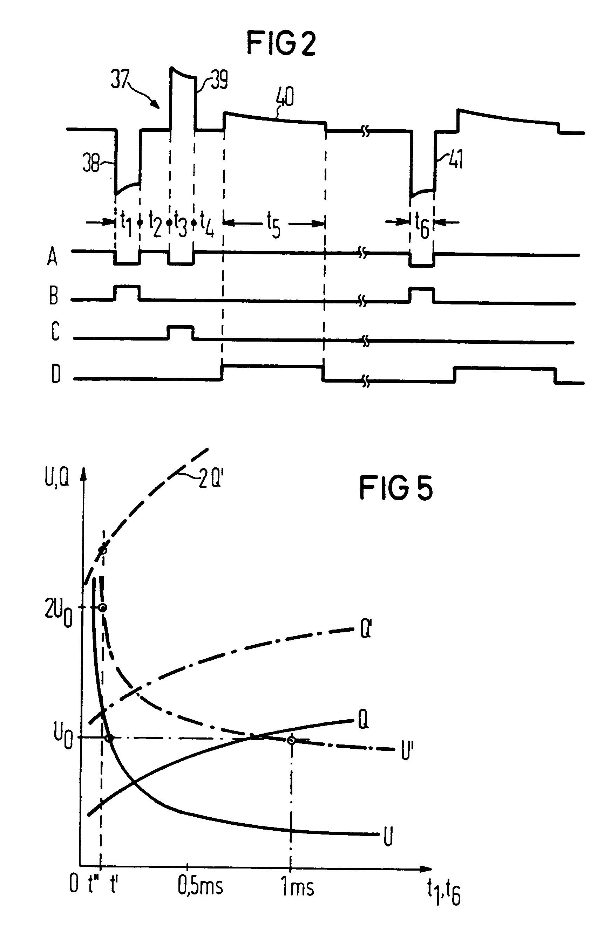

Innerhalb des Stimulationimpulsgenerators (1) ist ein Speicherkondensator (29) an seinen Anschlüssen (30,31) über ein erstes Schalterpaar A mit dem Ausgang (4) des Stimulationsspannungsgenerators (5) verbunden. Der Speicherkondensator (29) ist ferner über ein zweites Schalterpaar B und ein drittes Schalterpaar C, die in einer Brückenschaltung angeordnet sind, mit der indifferenten Elektrode (2) und über einen Ausgangskondensator (32) mit der Stimulationselektrode (3) verbunden. Je nachdem, ob das Schalterpaar B oder das Schalterpaar C schließt, wird der Speicherkondensator (29) an seinem mit (30) bezeichneten Anschluß mit der indifferenten Elektrode (2) und an seinem Anschluß (31) mit dem Ausgangskondensator (32) verbunden oder umgekehrt mit seinem Anschluß (31) an der indifferenten Elektrode (2) und mit dem Anschluß (30) an dem Ausgangskondensator (32) angeschlossen. Schließlich ist ein Schalter D vorgesehen, der die Stimulationselektrode (3) über den Ausgangskondensator (32) mit der indifferenten Elektrode (2) verbindet. Die Schalterpaare A, B und C und der Schalter D werden über ihnen zugeordnete Steuerleitungen (33,34,35,36) von der Zeitgebereinrichtung (25) einzeln angesteuert, wie dies im folgenden anhand von FIG 2 erläutert wird.

FIG 2 zeigt in einem Zeitdiagramm ein Beispiel für einen biphasischen Stimulationsimpuls (37), der aus einem negativen Teilimpuls (38) gefolgt von einem positiven Teilimpuls (39) und einem Schnellentladeimpuls (40) für den Ausgangskondensator (32) besteht; ferner sind ein monophasischer Stimulationsimpuls (41) sowie die zur Erzeugung der Stimulationsimpulse (37, 41) von der Zeitgebereinrichtung (25) an die Schalterpaare A, B und C sowie den Schalter D abgegebenen, hier entsprechend bezeichneten Steuersignale zu sehen. Dabei bedeutet ein von Null abweichender Signalzustand, daß der zugeordnete Schalter geschlossen ist.

Solange das Schalterpaar A geschlossen ist, wird der Speicherkondensator (29) auf die von der Steuereinrichtung (17) vorgegebene und von dem Stimulationsspannungsgenerator (5) erzeugte Ausgangsspannung aufgeladen. Dabei wird der Speicherkondensator (29) auf die Spannung U₀ der Batterie (6) aufgeladen, indem bei geöffneten Schalter (10,11) der Schalter (9) geschlossen wird. Um dagegen den Speicherkondensator (29) auf einen Wert zwischen der Spannung U₀ und der doppelten Batteriespannung (2)U₀ aufzuladen, wird der gewünschte Wert von der Steuereinrichtung (17) als Digitalwert über die zweite ausgangsseitige Steuerleitung (23) dem Digital-/Analog-Umsetzer (22) zugeführt, der den zweiten Eingang (21) des Komparators (19) mit dem entsprechenden analogen Spannungswert beaufschlagt. Gleichzeitig werden die Schalter (9,10) einerseits und der Schalter (11) andererseits mit hoher Folgefrequenz abwechselnd geschlossen und geöffnet, so daß elektrische Ladung aus der Batterie (6) portionsweise in den Speicherkondensator (29) gepumpt wird. Sobald die Spannung an dem Ausgang (4) des Stimulationspannungsgenerators (5) und damit an dem ersten Eingang (20) des Komparators (19) den Wert der Vergleichsspannung an dem zweiten Eingang (21) des Komparators (19) erreicht, schaltet dieser über die Steuerleitung (18) die Schalter (9,11) in einen geöffneten Zustand und unterbricht den Vorgang der Ladungspumpe. Das Prinzip der Spannungserhöhung ist auch aus der EP-A-0 026 481 bekannt, die auch weitere, im Zusammenhang mit der erfindungsgemäßen Anordnung anwendbare Möglichkeiten zur Spannungserhöhung aufzeigt.

Zur Erzeugung der biphasischen Stimulationsimpulses (37) zwischen der Stimulationselektrode (3) und der indifferenten Elektrode (2) wird bei geöffnetem Schalterpaar A das Schalterpaar B für eine Zeitdauer t₁ und nach einer anschließenden Pause t₂ das Schalterpaar C für eine Zeitdauer t₃ geschlossen; schließlich wird nach einer weiteren Pause t₄ der Schalter D für eine Zeitdauer t₅ geschlossen, wodurch der Ausgangskondensator (32) schnellentladen wird. Die Impulsdauern t₁ und t₃ und die Impulsamplituden der Teilimpulse (38,39) sind jeweils im wesentlichen gleich, wobei die Impulsdauern t₁ und t₃ vorzugsweise im Bereich zwischen 0,03 ms und 1 ms variiert werden. Die Impulspause t₂ zwischen den beiden Teilimpulsen (38,39) dauert etwa 1 ms und die Dauer des Schnellentladeimpulses (40), der sich auch ohne Pause t₄ direkt an den positiven Teilimpuls (39) anschließen kann, beträgt etwa 4 ms. Die angegebenen Werte sind lediglich Richtwerte und variierbar, wobei aber vorzugsweise spätestens nach 6 bis 7 ms der gesamte biphasische Stimulationsimpuls (37) beendet ist und die Reaktion des stimulierten Gewebes mittels der an die Stimulationselektrode (3) angeschlossenen Detektoreinrichtung (26) erfaßt wird. Zu diesem Zeitpunkt ist die durch den stimulierenden negativen Teilimpuls (38) erzeugte Polarisation im Gewebe auch bei relativ großer Stimulationsenergie auf einige mV abgesunken, so daß eine eindeutige, von überlagerungen durch die Polarisationserscheinungen weitestgehend freie Detektion der Reaktion des Gewebes ermöglicht ist. Durch die Impulspause t₂ zwischen den beiden Teilimpulsen (38,39) wird verhindert, daß die stimulationsauslösende Wirkung des ersten Teilimpulses (38) durch den nachfolgenden Teilimpuls (39) reduziert wird, wobei mit zunehmender Impulspause t₂ sogar eine leichte Erhöhung der stimulationsauslösenden Wirkung festgestellt werden konnte.

Wird die Reaktion des stimulierten Gewebes über die der Stimulationselektrode (3) benachbarte Meßelektrode (28) erfaßt, so kann anstelle des biphasischen Stimulationsimpulses (37) der monophasische Stimulationsimpuls (41) zur Gewebestimulation verwendet werden, der nur die Hälfte des Stromverbrauches aufweist. Dies liegt daran, daß das Aktionspotential im Gewebe mit einer Zeitverzögerung von der Stimulationselektrode (3) kommend zur Meßelektrode (28) gelangt, so daß in dieser Zeit die Polarisationserscheinungen weitestgehend abgeklungen sind.

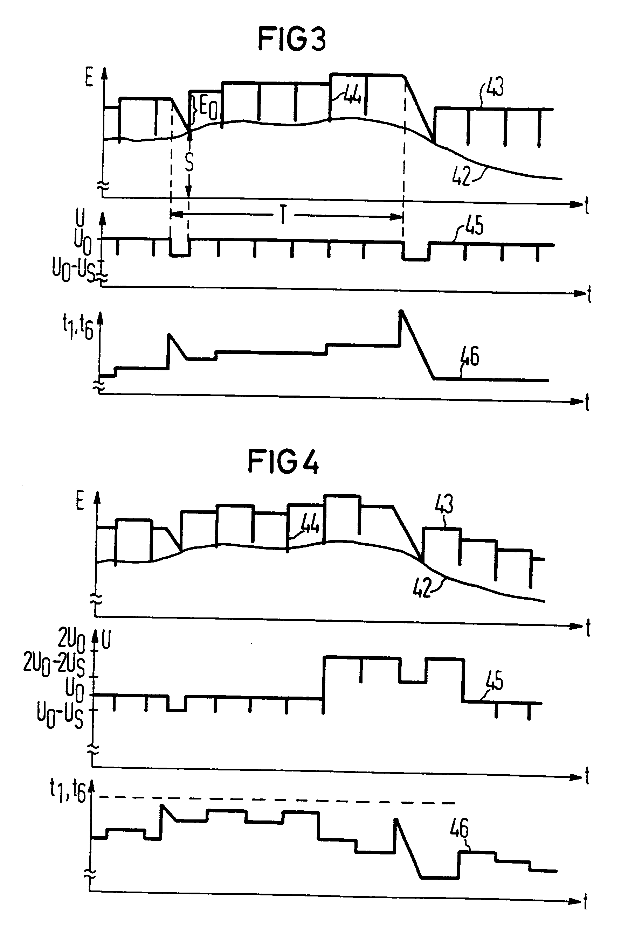

Der mit (41) bezeichnete monophasische Stimulationsimpuls wird durch Schließen des Schalterpaares B erzeugt; anschließend wird der Ausgangskondensator (32) durch Schließen des Schalters D mit einem Schnellladeimpuls entladen. Wie FIG 2 zeigt, ist der Energieinhalt des monophasischen Stimulationsimpulses (41) bei gleicher Impulsamplitude und Impulsdauer t₆ = t₁ = t₃ um die Hälfte geringer, als der des biphasischen Stimulationsimpulses (37). Beide Stimulationsimpulse (37,41) zeigen jedoch trotz ihres unterschiedlichen Energieinhaltes bei der Gewebestimulation etwa die gleiche stimulationsauslösende Wirkung. Daher werden die biphasischen Stimulationsimpulse (37) nur dann erzeugt, wenn die Reaktion des Gewebes auf die Stimulation über die Stimulationselektrode (3) erfaßt werden soll; ansonsten werden zur Gewebestimulation monophasische Stimulationsimpulse entsprechend dem Impuls (41) erzeugt. Im folgenden wird dies anhand der FIG 3 und 4 erläutert.

In den FIG 3 und 4 ist jeweils in dem oberen Zeitdiagramm ein mit (42) bezeichneter möglicher Verlauf der Stimulationsempfindlichkeit des zu stimulierenden Gewebes in Abhängigkeit von der Zeit t dargestellt. Die Stimulationsempfindlichkeit wird durch einen Energiewert E (oder eine elektrische Spannung oder einen Strom) ausgedrückt, den ein Stimulationsimpuls mindestens für eine erfolgreiche Stimulation des Gewebes aufweisen muß. Um jederzeit eine erfolgreiche Stimulation sicherzustellen, könnte das Gewebe ständig mit einer maximalen Energie stimuliert werden. Der Energiebedarf wäre in diesem Falle jedoch sehr hoch. Daher wird die Stimulationsenergie entsprechend dem Verlauf der mit (43) bezeichneten Kurve an die veränderliche Stimulationsempfindlichkeit (42) des Gewebes angepaßt. Dazu wird in periodischen Zeitabständen T die jeweils aktuelle Stimulationsempfindlichkeit des Gewebes ermittelt, indem das Gewebe mit im Falle des Anschlusses der Detektoreinrichtung (26) an die Stimulationselektrode (3) biphasischen, anderenfalls monophasischen Stimulationsimpulsen (37,41 vgl. FIG 2) stimuliert wird und gleichzeitig die Reaktion des Gewebes auf die Stimulation mittels der Detektoreinrichtung (26) erfaßt wird. Solange jedesmal eine Reaktion des Gewebes detektiert wird, wird die Stimulationsenergie des jeweils nächsten biphasischen bzw. monophasischen Stimulationsimpulses verringert, bis eine Reaktion des Gewebes ausbleibt, weil die aktuelle Stimulationsempfindlichkeit S unterschritten wurde. Die Stimulationsempfindlichkeit S entspricht dabei der aktuell minimal erforderlichen Stimulationsenergie, um eine Stimulation überhaupt auslösen zu können. Nach Ermittlung der Stimulationsempfindlichkeit S werden zur weiteren Stimulation des Gewebes monophasische Stimulationsimpulse (41) mit einer um einen Sicherheitswert E₀ über der soeben ermittelten Stimulationsempfindlichkeit S liegenden Energie E = S + E₀ erzeugt, bis nach Ablauf des Zeitabstandes T die Stimulationsempfindlichkeit (42) des Gewebes auf die oben beschriebene Weise erneut ermittelt wird. Der Sicherheitswert E₀ kann durch einen Festwert oder einen prozentualen Anteil des gefundenen Wertes S für die Stimulationsempfindlichkeit (42) definiert werden. Bei dem Kurvenverlauf (43) ist die Energie der verwendeten Stimulationsimpulse auf den stimulationsrelevanten Anteil normiert; d.h. von den biphasischen Stimulationsimpulsen (37) ist nur die Energie des ersten stimulationsauslösenden Teilimpulses (38) berücksichtigt, so daß der tatsächliche Energieaufwand bei der biphasischen Stimulation etwa doppelt so hoch ist, wie bei der monophasischen Stimulation. Um einerseits eine schnelle Anpassung der Stimulationsenergie (43) an den Verlauf (42) der Stimulationsempfindlichkeit zu erreichen, andererseits aber die Häufung der bei Verwendung von biphasischen Stimulationsimpulsen (37) relativ energieaufwendigen Erfassung der Stimulationsempfindlichkeit möglichst gering zu halten, wird der Zeitabstand T bis zur jeweils nächsten Ermittlung der Stimulationsempfindlichkeit in Abhängigkeit von deren Änderung zwischen der aktuellen und der vorangegangenen Ermittlung variiert, wobei bei größer werdender Änderung der Zeitabstand T verringert wird.

Zwischen jeweils zwei Vorgängen zur Ermittlung der Stimulationsempfindlichkeit wird periodisch, beispielsweise nach jedem 10. monophasischen Stimulationsimpuls (41) ein einzelner biphasischer, oder im Falle des Anschlusses der Detektoreinrichtung (26) an die Meßelektrode (28) ein einzelner monophasischer Stimulationsimpuls (44) mit einer um den Sicherheitswert E₀ verringerten Energie erzeugt und mittels der Detektoreinrichtung (26) überprüft, ob die so verminderte Stimulationsenergie nach wie vor über der Stimulationsempfindlichkeit (42) liegt. Ist dies nicht der Fall, so wird die Stimulationsenergie für die folgenden monophasischen Stimulationsimpulse um einen den Sicherheitswert E₀ übersteigenden Betrag erhöht. Abweichend von FIG 3 zeigt FIG 4 ein Ausführungsbeispiel, bei dem zusätzlich für den Fall, daß die um den Sicherheitswert E₀ verringerte Stimulationsenergie (43) die Stimulationsempfindlichkeit (42) übersteigt, die Stimulationsenergie für die nächsten monophasischen Stimulationsimpulse auf einen geringeren Betrag, als der des Sicherheitswertes E₀ erhöht wird. Zur periodischen Erzeugung der Einzel-Stimulationsimpulse (44) enthält die in FIG 1 gezeigte Steuereinrichtung (17) einen dort nicht dargestellten Soft- oder Hardware-Zähler, der die monophasischen Stimulationsimpulse (41) zählt und beim Erreichen eines vorgegebenen Zählerstandes (hier 10) zurückgesetzt wird und die Abgabe des Einzel-Stimulationsimpulses (44) veranlaßt.

Die jeweils unteren Zeitdiagramme in den FIG 3 und 4 zeigen, in welcher Weise der Verlauf der Stimulationsener2gie (43) der Stimulationsimpulse durch Änderung der Impulsamplitude U und Impulsdauer t₁, t₆ eingestellt wird. Dabei zeigt der in FIG 3 mit (45) bezeichnete Verlauf der Impulsamplitude, daß diese mit Ausnahme der Zeiten, in denen eine Detektion der Reaktion des Gewebes stattfindet, ständig der verfügbaren Batteriespannung U₀ entspricht. Lediglich in den Zeiten, in denen gleichzeitig mit der Erzeugung der Stimulationsimpulse auch die Reaktion des Gewebes auf die Stimulation überwacht wird, wird die Impulsamplitude U um einen dem Sicherheitswert E₀ entsprechenden Spannungswert U_{S} verringert. Im übrigen erfolgt die Änderung der Stimulationsenergie (43) durch Variation der Impulsdauer t₁ bzw. t₆ der Stimulationsimpulse entsprechend der mit (46) bezeichneten Kurve. FIG 4 zeigt ferner, daß dann, wenn die Impulsdauer t₁, t₆ einen maximalen, hier durch eine gestichelte Linie angedeuteten Wert zu übersteigen droht, eine Verdoppelung der Stimulationsamplitude U auf 2U₀ erfolgt. Auch hierbei werden im Zusammenhang mit der Detektion der Stimulationsempfindlichkeit Stimulationsimpulse mit einer verminderten Impulsamplitude U erzeugt, wobei die Verminderung vorzugsweise 2U_{S} beträgt, um einen von der Impulsamplitude U_{S} unabhängigen konstanten Sicherheitswert E₀ zu erhalten. Die Verdoppelung der Impulsamplitude wird darüberhinaus auch anhand von FIG 5 erläuter. Die FIG 3 und 4 zeigen also, daß bei der Ermittlung der Stimulationsempfindlichkeit (42) die Stimulationsimpulse mit einer Impulsamplitude erzeugt werden, deren Spannung U um einen dem Sicherheitswert E₀ entsprechenden Spannungswert U_{S} unter der maximal verfügbaren Spannung U₀ der Spannungsquelle (6) liegt, wobei zur Veränderung der Stimulationsenergie (43) die Impulsdauer t₁, t₆ der Stimulationsimpulse verändert wird, und daß im Anschluß an die Ermittlung der Stimulationsempfindlichkeit S die Stimulationsimpulse mit einer der verfügbaren Spannung U₀ der Spannungsquelle (6) entsprechenden Impulsamplitude erzeugt werden. Daher liegt außerhalb der Zeiten, in denen die Stimulationsempfindlichkeit des Gewebes erfaßt wird, die Impulsamplitude in vorteilhafter Weise um den als elektrische Spannung, d.h. in Volt ausgedrückten Sicherheitswert U_{S} über der zuletzt ermittelten Reizschwelle S des zu stimulierenden Gewebes, so daß hiermit für den behandelnden Arzt eine sichere Information über den Sicherheitswert E₀ bzw. U_{S} gegeben ist. Dadurch, daß in der Zeitdauer T zwischen jeweils zwei Vorgängen zur Ermittlung der Stimulationsempfindlichkeit die Stimulationsimpulse mit einer der verfügbaren Spannung U₀ der Spannungsquelle (6) entsprechenden Impulsamplitude erzeugt werden, ist, wie im folgenden anhand von FIG 5 erläutert wird, die Strom- bzw. Ladungsentnahme aus der Spannungsquelle (6) so gering wie möglich.

FIG 5 zeigt in einem Diagramm für eine bestimmte Stimulationsempfindlichkeit des Gewebes die zur Stimulation mindestens erforderliche Spannung (Impulsamplitude) U und Ladung Q eines Stimulationsimpulses in Abhängigkeit von dessen Impulsdauer, für die im Falle des biphasischen Stimulationsimpulses (37 vgl. FIG 2) die Impulsdauer t₁ des ersten stimulierenden Teilimpulses (38) maßgeblich ist und die im Falle des monophasischen Stimulationsimpulses (41) dessen Impulsdauer t₆ entspricht. Bei dem biphasischen Stimulationsimpuls (37) ist die Ladung aufgrund des zweiten Teilimpulses (39) insgesamt doppelt so groß, so daß der Kurvenverlauf Q im Vergleich zu dem monophasischen Impuls (41) nur die Hälfte der gesamten Ladung des biphasischen Stimulationsimpulses (37) wiedergibt.

Wie zu erkennen ist, nehmen die Werte für die Spannung U mit geringer werdender Impulsdauer t₁, t₆ zu, während gleichzeitig die Ladung Q, die dem Produkt aus der Stromstärke und der Impulsdauer t₁, t₆ des Stimulationsimpulses entspricht, abnimmt. Bei abnehmender Stimulationsempfindlichkeit des Gewebes verschieben sich die Kurven Q und U nach oben, wie dies durch die Kurvenzüge Q′ und U′ angegeben ist. Steht, wie im Falle der Batterie (6, vgl. FIG 1), eine bestimmte Spannung U₀ z.B. 2,5 V zur Verfügung, so ist bei der durch die Kurvenverläufe Q und U definierten Stimulationsempfindlichkeit der Funktionsverlauf der Ladung Q für die Bestimmung der Ladungsentnahme aus der Batterie (6), d.h. des Stromverbrauchs maßgeblich. Wie FIG 5 zeigt, ist der Stromverbrauch (d.h. die Ladungsentnahme) dann minimal, wenn die ganze zur Verfügung stehende Spannung U₀ zur Erzeugung des Stimulationsimpulses herangezogen wird; dabei ergibt sich eine Impulsdauer t′. Nimmt die Stimulationsempfindlichkeit des Gewebes ab, wie dies durch die Spannungskurve U′ und die zugehörige Ladungskurve Q′ verdeutlicht ist, so ist zur erfolgreichen Stimulation eine Erhöhung der Stimulationsenergie erforderlich. Dazu wird bei gleichbleibender Stimulationsspannung U₀ die Impulsdauer t₁, t₆ der Stimulationsimpulse erhöht, bis ein Wert von etwa 1 ms erreicht wird. Über diesen Wert hinaus würde die gesamte Dauer bei dem bipolaren Stimulationsimpuls (37) für eine Detektion der Reaktion des Gewebes auf die Stimulation zu lang werden, weswegen zur weiteren Erhöhung der Stimulationsenergie mittels der Spannungsverdoppelungsschaltung (7 vgl. FIG 1) eine doppelte Batteriespannung 2 U₀ eingestellt wird, wodurch sich eine Reduzierung der Impulsdauer t₁ auf t₂′ ergibt. Allerdings ist mit der Spannungsverdoppelung auch eine Verdoppelung des Batteriestromes, also der Ladungsentnahme aus der Batterie (6) verbunden, was durch die Ladungskurve 2 Q′ verdeutlicht wird. Wie FIG 5 zeigt, wird diese Verdoppelung der Ladungsentnahme aus der Batterie (6) nur teilweise dadurch reduziert, daß bei der auf 2 U₀ erhöhten Impulsamplitude eine geringere Impulsdauer t₂′ zur Stimulation erforderlich ist. Daher wird zur Reduzierung der Ladungsentnahme aus der Batterie (6) die Spannungsverdoppelung erst dann aktiviert, wenn bei der Stimulation mit der verfügbaren Batteriespannung U₀ die Impulsdauer t₁, t₆ einen vorgegebenen Maximalwert (hier 1 ms) überschreitet.

## Patentansprüche

1. Verfahren zum Betreiben einer Anordnung zur Gewebestimulation bei einem Lebewesen mit einem aus einer Spannungsquelle (6) gespeisten Stimulationsimpulsgenerator (1) zur Erzeugung von Stimulationsimpulsen (37, 41), mit einer Detektoreinrichtung (26) zur Erfassung der Reaktion des Gewebes auf die Stimulation und mit einer den Stimulationsimpulsgenerator (1) steuernden Steuereinrichtung (17), die zur Ermittlung der Stimulationsempfindlichkeit (42) des Gewebes solange eine Veränderung der Energie E nacheinander erzeugter Stimulationsimpulse (37,41) veranlaßt, bis die Detektoreinrichtung (26) einen Wechsel von einem Ausbleiben der Reaktion zu einer Reaktion oder umgekehrt erfaßt, wobei anschließend die Stimulationsimpulse (37,41) mit einer Energie erzeugt werden, die dem für die Stimulationsempfindlichkeit (42) ermittelten Wert S zuzüglich einem Sicherheitswert E₀ entspricht, **dadurch gekennzeichnet**, daß bei der Ermittlung der Stimulationsempfindlichkeit (42) die Stimulationsimpulse (37,41) mit einer Impulsamplitude erzeugt werden, deren Spannung U um einen dem Sicherheitswert E₀ entsprechenden Spannungswert U_{S} unter der maximal verfügbaren Spannung U₀ der Spannungsquelle (6) liegt, wobei zur Veränderung der Stimulationsenergie E die Impulsdauer t₁, t₆ der Stimulationsimpulse (37,41) verändert wird, und daß im Anschluß an die Ermittlung der Stimulationsempfindlichkeit (42) die Stimulationsimpulse (37, 41) mit einer der verfügbaren Spannung U₀ der Spannungsquelle (6) entsprechenden Impulsamplitude und der bei der Detektion des Wechsels bei der Reaktion des Gewebes eingestellten Impulsdauer erzeugt werden.

2. Verfahren nach Anspruch 1, **dadurch gekennzeichnet**, daß nach der Ermittlung der Stimulationsempfindlichkeit (42) bis zur nächsten Ermittlung in sich wiederholenden vorgegebenen Zeitabständen einzelne Stimulationsimpulse (44) mit um den Sicherheitswert U_{S} verringerter Impulsamplitude U erzeugt werden, daß dabei die Reaktion des Gewebes mittels der Detektoreinrichtung (26) detektiert wird und daß bei einem Ausbleiben der Reaktion für die nächsten Stimulationsimpulse die Impulsdauer (t₁, t₆) erhöht und die Impulsamplitude U auf den der verfügbaren Spannung U₀ entsprechenden Wert zurückgestellt wird.

3. Verfahren nach Anspruch 2, **dadurch gekennzeichnet**, daß bei Detektion einer Reaktion des Gewebes auf den jeweiligen einzelnen Stimulationsimpuls (244) die Impulsamplitude U für die nächsten Stimulationsimpulse bei unveränderter Impulsdauer auf den der verfügbaren Spannung U₀ entsprechenden Wert zurückgestellt wird.

4. Verfahren nach Anspruch 2, **dadurch** **gekennzeichnet**, daß bei Detektion einer Reaktion des Gewebes auf den jeweils einzelnen Stimulationsimpuls (44) die Impulsdauer t₁, t₆ für die nächsten Stimulationsimpulse vermindert und die Impulsamplitude auf den der verfügbaren Spannung U₀ entsprechenden Wert zurückgestellt wird.

5. Verfahren nach einem der vorangehenden Ansprüche, **dadurch gekennzeichnet**, daß bei einem Anschluß des Stimulationsimpulsgenerators (1) und der Detektoreinrichtung (26) an einer gemeinsamen Stimulationselektrode (3) die in Verbindung mit einer nachfolgenden Detektion der Reaktion des Gewebes zu erzeugenden Stimulationsimpulse in Form von biphasischen Stimulationsimpulsen (37) erzeugt werden und daß alle übrigen Stimulationsimpulse in Form von monophasischen Impulsen (41) erzeugt werden.

6. Verfahren nach Anspruch 5, **dadurch gekennzeichnet**, daß jeder biphasische Stimulationsimpuls (37) aus Teilimpulsen (38,39) unterschiedlicher Polarität mit einer zwischen den Teilimpulsen (38,39) liegenden Impulspause t₂ besteht und daß die Teilimpulse (38,39) jeweils eine weitgehend gleiche Impulsamplitude und Impulsdauer t₁, t₃ aufweisen.

7. Verfahren nach einem der vorangehenden Ansprüche, **da****durch gekennzeichnet**, daß zur Veränderung der Energie E der Stimulationsimpulse (37,41), deren Impulsdauer t₁, t₆ ausgehend von der durch die verfügbare Spannung U₀ der Spannungsquelle (6) vorgegebenen Impulsamplitude U zwischen einem Minimalwert und einem Maximalwert verändert wird und daß beim Erreichen des Maximalwertes zur Einstellung einer noch höheren Energie die Impulsamplitude U auf einen durch Vervielfachung der Spannung U₀ der Spannungsquelle (6) erhöhten Wert eingestellt wird.

## Claims

1. Method of operating a tissue-stimulating device in a living organism, having a stimulation pulse generator (1), powered from a voltage source (6), to generate stimulation pulses (37,41), having a detector device (26) to pick up the reaction of the tissue to the stimulation and having a control device (17) which controls the stimulation pulse generator (1) and which, to determine the stimulation sensitivity (42) of the tissue, causes a change of the energy E of successively generated stimulation pulses (37,41) until such a time as the detector device (26) picks up a change from an absence of reaction to a reaction or vice versa, in which subsequently the stimulation pulses (37,41) are generated with an energy which corresponds to the value S determined for the stimulation sensitivity (42) plus a safety value Eₒ, characterized in that in the determination of the stimulation sensitivity (42), the stimulation pulses (37,41) are generated with a pulse amplitude, the voltage U of which is less, by a voltage value Uₛ corresponding to the safety value Eₒ, than the maximum available voltage Uₒ of the voltage source (6), in which, in order to change the stimulation energy E, the pulse duration t₁, t₆ of the stimulation pulses (37,41) is changed, and in that, subsequently to the determination of the stimulation sensitivity (42), the stimulation pulses (37,41) are generated with a pulse amplitude corresponding to the available voltage Uₒ of the voltage source (6) and with the pulse duration set upon the detection of the change in the reaction of the tissue.

2. Method according to Claim 1, characterized in that, after the determination of the stimulation sensitivity (42) until the next determination, individual stimulation pulses (44) are generated at repeating prescribed time intervals with a pulse amplitude U reduced by the safety value Uₛ, in that in this case the reaction of the tissue is detected by means of the detector device (26), and in that in the case of an absence of reaction for the next stimulation pulses the pulse duration t₁, t₆) is increased and the pulse amplitude U is reset to the value corresponding to the available voltage Uₒ.

3. Method according to Claim 2, characterized in that in the case of detection of a reaction of the tissue to the respective individual stimulation pulse (244) the pulse amplitude U for the next stimulation pulses is reset, with unchanged pulse duration, to the value corresponding to the available voltage Uₒ.

4. Method according to Claim 2, characterized in that in the case of detection of a reaction of the tissue to the respective individual stimulation pulse (44) the pulse duration t₁, t₆ for the next stimulation pulses is reduced and the pulse amplitude is reset to the value corresponding to the available voltage Uₒ.

5. Method according to one of the preceding claims, characterized in that in the case of a connection of the stimulation pulse generator (1) and the detector device (26) to a common stimulation electrode (3) the stimulation pulses to be generated in conjunction with a subsequent detection of the reaction of the tissue are generated in the form of biphasic stimulation pulses (37), and in that all remaining stimulation pulses are generated in the form of monophasic pulses (41).

6. Method according to Claim 5, characterized in that each biphasic stimulation pulse (37) comprises partial pulses (38,39) of differing polarity with a pulse pause t₂ situated between the partial pulses (38,39), and in that the partial pulses (38,39) exhibit in each instance a substantially equal pulse amplitude and pulse duration t₁, t₃.

7. Method according to one of the preceding claims, characterized in that to change the energy E of the stimulation pulses (37,41), their pulse duration t₁, t₆ is changed between a minimum value and a maximum value, starting from the pulse amplitude U predetermined by the available voltage Uₒ of the voltage source (6), and in that, upon reaching the maximum value, to set an even higher energy, the pulse amplitude U is set to a value increased by multiplication of the voltage Uₒ of the voltage source (6).

## Revendications

1. Procédé pour faire fonctionner un dispositif destiné à la stimulation du tissu d'un être vivant, comportant un générateur (1) d'impulsions de stimulation alimenté par une source de tension (6) et destiné à produire des impulsions de stimulation (37, 41), un dispositif de détection (26) destiné à détecter la réaction du tissu à la stimulation et un dispositif de commande (17), qui commande le générateur (1) d'impulsions de stimulation et qui, pour la détermination de la sensibilité (42) du tissu à la stimulation, provoque une modification de l'énergie E d'impulsions de stimulation (37, 41) produites successivement, jusqu'à ce que le dispositif de détection (26) détecte le passage d'une absence de réaction à une réaction ou l'inverse, les impulsions de stimulation (37, 41) étant ensuite produites avec une énergie qui correspond à la valeur S, déterminée pour la sensibilité (42) à la stimulation, plus une valeur de sécurité E₀, caractérisé en ce qu'il consiste à produire, lors de la détermination de la sensibilité (42) à la stimulation, les impulsions de stimulation (37, 41) avec une amplitude, dont la tension U est inférieure, d'une valeur de tension U_{S} qui correspond à la valeur de sécurité E₀, à la tension maximale disponible U₀ de la source de tension (6), en modifiant, pour modifier l'énergie de stimulation E, la durée t₁, t₆ des impulsions de stimulation (37, 41), et après avoir déterminé la sensibilité (42) à la stimulation, à produire les impulsions de stimulation (37, 41) avec une amplitude qui correspond à la tension disponible U₀ de la source de tension (6) et avec la durée d'impulsion établie lors de la détection du changement pour la réaction du tissu.

2. Procédé suivant la revendication 1, caractérisé en ce qu'il consiste, après la détermination de la sensibilité (42) à la stimulation, à produire, jusqu'à la détermination immédiatement suivante, des impulsions individuelles de stimulation (44) d'une amplitude U réduite de la valeur de sécurité U_{S} pendant des intervalles de temps qui se répètent, à détecter la réaction du tissu à l'aide du dispositif de détection (26) et, en l'absence de réaction, à augmenter la durée (t₁, t₆) des impulsions de stimulation immédiatement suivantes et à en ramener l'amplitude (U) à la valeur qui correspond à la tension disponible U₀.

3. Procédé suivant la revendication 2, caractérisé en ce qu'il consiste à ramener, après détection d'une réaction du tissu à l'impulsion individuelle de stimulation (244), l'amplitude U des impulsions de stimulation immédiatement suivantes à la valeur qui correspond à la tension U₀ disponible, sans modifier la durée des impulsions.

4. Procédé suivant la revendication 2, caractérisé en ce qu'il consiste à diminuer, après détection d'une réaction du tissu à l'impulsion individuelle de stimulation (44), la durée t₁, t₆ des impulsions de stimulation immédiatement suivantes et à en ramener l'amplitude à la valeur qui correspond à la tension disponible U₀.

5. Procédé suivant l'une des revendications précédentes, caractérisé en ce qu'il consiste, lorsque le générateur (1) d'impulsions de stimulation (1) et le dispositif de détection (26) sont raccordés à une électrode (3) commune de stimulation, à produire les impulsions de stimulation, qui doivent être produites en liaison avec une détection ultérieure de la réaction du tissu, sous la forme d'impulsions de stimulation (37) biphasées, et à produire toutes les autres impulsions de stimulation sous la forme d'impulsions (41) monophasées.

6. Procédé suivant la revendication 5, caractérisé en ce que chaque impulsion de stimulation (37) biphasée est constituée d'impulsions partielles (38, 39) de polarité différente, avec une pause t₂ entre les impulsions partielles (38, 39), et les impulsions partielles (38, 39) ont dans une grande mesure une amplitude et une durée t₁, t₃ identiques.

7. Procédé suivant l'une des revendications précédentes, caractérisé en ce qu'il consiste, pour modifier l'énergie E des impulsions de stimulation (37, 41), à modifier leur durée t₁, t₆ à partir de l'amplitude U des impulsions, prescrite par la tension U₀ disponible de la source de tension (6), entre une valeur minimale et une valeur maximale et lorsque la valeur maximale est atteinte, pour établir une énergie encore plus grande, à donner a l'amplitude U des impulsions une valeur plus grande en multipliant la tension U₀ de la source de tension (6).
